# EUROPEAN PATENT SPECIFICATION

(11) **EP 2 774 564 B1**
(45) Date of publication and mention of the grant of the patent: **25.05.2016**
(21) Application number: 14158142.1
(22) Date of filing: 06.03.2014
(51) Int. Cl.: A61B 17/70

(54) **Percutaneous rod inserter**
Perkutaner Stabeinführer
Dispositif d'insertion de tige percutanée

(30) Priority: 06.03.2013 US 201313786798
(43) Date of publication of application: 10.09.2014
(73) Proprietor: Alphatec Spine, Inc., Carlsbad, CA 92008 (US)
(72) Inventor: Meyer, Nathan, Vista, CA California 92083 (US); Loquet, Richard, Hemet, CA California 92545 (US); Parikh, Anand, San Diego, CA California 92101 (US); Hutton, Clark, Oceanside, CA 92008 (US)
(74) Representative: Giles, Ashley Simon

(56) References cited:
- US-A1- 2008 077 138
- US-A1- 2009 318 954
- US-A1- 2010 036 443
- US-A1- 2011 152 942

## Description

### FIELD

The present disclosure generally relates to the field of spinal orthopedics, and more particularly to an instrumented for percutaneously inserting a spinal rod.

### BACKGROUND

The spine is a flexible column formed of a plurality of bones called vertebrae. The vertebrae are hollow and piled one upon the other, forming a strong hollow column for support of the cranium and trunk. The hollow core of the spine houses and protects the nerves of the spinal cord. The different vertebrae are connected to one another by means of articular processes and intervertebral, fibrocartilaginous bodies. Various spinal disorders may cause the spine to become misaligned, curved, and/or twisted or result in fractured and/or compressed vertebrae. It is often necessary to surgically correct these spinal disorders.

The spine includes seven cervical (neck) vertebrae, twelve thoracic (chest) vertebrae, five lumbar (lower back) vertebrae, and the fused vertebrae in the sacrum and coccyx that help to form the hip region. While the shapes of individual vertebrae differ among these regions, each is essentially a short hollow shaft containing the bundle of nerves known as the spinal cord. Individual nerves, such as those carrying messages to the arms or legs, enter and exit the spinal cord through gaps between vertebrae.

The spinal disks act as shock absorbers, cushioning the spine, and preventing individual bones from contacting each other. Disks also help to hold the vertebrae together. The weight of the upper body is transferred through the spine to the hips and the legs. The spine is held upright through the work of the back muscles, which are attached to the vertebrae. While the normal spine has no side-to-side curve, it does have a series of front-to-back curves, giving it a gentle "S" shape. If the proper shaping and/or curvature are not present due to scoliosis, neuromuscular disease, cerebral palsy, or other disorder, it may be necessary to straighten or adjust the spine into a proper curvature.

Generally the correct curvature is obtained by manipulating the vertebrae into their proper position and securing that position with a rigid system of screws and rods. The screws may be inserted into the pedicles of the vertebrae to act as bone anchors, and the rods may be inserted into heads of the screws. Two rods may run substantially parallel to the spine and secure the spine in the desired shape and curvature. Thus the rods, which are shaped to mimic the correct spinal curvature, force the spine into proper alignment. Bone grafts are then placed between the vertebrae and aid in fusion of the individual vertebrae together to form a correctly aligned spine.

Various inserter instruments may be used to insert the rods in a minimally invasive surgical procedure. These inserter instruments may include different styles including straight, curved, and central grasping for multiple angles of approach. One particular instrument includes a curved profile and end-grasping tip that engages with an end of the rod. The end-grasping tip requires significant force to grasp the end of the rod while maneuvering the rod percutaneously into place within two or more heads of prior inserted pedicle screws. The present disclosure addresses one or more problems associated with application of sufficient force to maintain control over the rod during surgery.

Examples of instruments for grasping an end of a spinal rod can be found in US 2010/0036443, US 2011/0152942 and US 2011/0106187.

### SUMMARY

The present invention relates to instrument for grasping an end of a spinal rod and percutaneous insertion of a spinal rod as claimed hereafter. Preferred embodiments of the invention are set forth in the dependent claims. According to the invention, there is provided an instrument for grasping an end of a spinal rod and percutaneous insertion of the spinal rod includes a curved housing, an immovable lip, a first slot, a pivoting lip and a slidable rod. The curved housing extends from a proximal end to a distal end and includes an interior channel. The immovable lip extends from the distal end and the first slot is in a side wall of the distal end. The pivoting lip is rotatably coupled to the distal end opposite the immovable lip. The slidable rod is disposed within the interior channel and slidably coupled at a first end to the first slot and a second slot of the pivoting lip, such that the slidable rod is translatable within the interior channel to position the pivoting lip relative to the immovable lip to hold a spinal rod therebetween.

Preferably, the instrument further comprises a threaded mechanism within a portion of the proximal end, rotatably engaged with a second end of the slidable rod and arranged to translate the slidable rod within the interior channel. In some embodiments, at least one of the immovable lip and the pivoting lip includes at least one mating feature configured to interact with at least one corresponding mating feature disposed on a spinal rod to further secure the rod. Suitably, the at least one mating feature of the at least one of the immovable lip and the pivoting lip comprises a male feature for engaging a corresponding female feature of a spinal rod. The immovable lip and the pivoting lip can be positioned to hold the spinal rod at a predetermined angle with respect to the curved housing. Conveniently, the instrument is configured to have a stopper mechanism for preventing unwanted excessive translation of the sliding rod. In some embodiments, the curved housing includes a groove and the slidable rod includes a corresponding protrusion configured to translate inside the groove to prevent excessive translation of the sliding rod.

Conveniently, the instrument includes a rotatable knob coupled to the threading mechanism. The pivoting lip and the immovable lip may be comprised within a rod holding mechanism. Preferably the instrument is configured such that rotation of the knob in one direction translates the slidable rod toward the distal end of the curved housing to secure a rod in the rod holding mechanism or between the pivoting lip and the immovable lip. Preferably the instrument is further configured such that rotation of the knob in another direction is configured to translate the slidable rod away from the distal end of the curved housing to release the rod. The one and another directions may be clockwise and counter-clockwise or vice versa.

Suitably, the threaded mechanism may include a first shaft rotatably coupled with the curved housing and a second shaft in threaded engagement with the first shaft. Conveniently, a portion of the threaded mechanism may rotate relative to the slidable rod. Preferably, a guide pin may extend through the first slot and the second slot to guide the movement of the pivoting lip as the slidable rod translates within the housing. In some embodiments, the distal end may comprise a third slot in an opposite side wall to the first slot and the guide pin may extend through the third slot. Suitably, a pivot pin extends through the pivoting lip and the distal end to rotatably couple the pivoting lip to the distal end. In some embodiments, the distal end of the curved housing may comprise an opening through which a portion of the pivoting lip projects in some positions.

### BRIEF DESCRIPTION OF THE DRAWINGS

FIGS. 1A-1E illustrate an exemplary percutaneous end-holding rod inserter tool according to the principles of the present disclosure.
FIG. 2 illustrates another exemplary percutaneous rod inserter according to the principles of the present disclosure.
FIG. 3 is an exploded perspective view of the rod inserter of FIG. 2.
FIG. 4 is a side view of the rod inserter of FIG. 2.
FIG. 5 is a front view of the rod inserter of FIG. 2.
FIGS. 6A-6D are cross-sectional views of the rod inserter of FIG. 2 in a plane VI as shown in FIG. 5 illustrating positioning of a jaw piece for grasping a spinal rod.
FIGS. 7A-7C are enlarged views of the distal end of the rod inserter of FIG. illustrating positioning of the jaw piece for grasping a spinal rod.

### DETAILED DESCRIPTION

The instrument of the present disclosure includes improvements over prior percutaneous end-holding rod inserters that increase grasping forces on spinal rods and improve maneuverability of the spinal rod within the surgical area of a patient. The instrument of the present disclosure includes a jaw piece that rotates about a distal end of the instrument to receive and grasp a spinal rod. Slots in the distal end of the instrument and in the jaw piece increase the moment of force applied to the jaw piece. The increased moment permits a surgeon to easily open and close the device as well as increasing the grasping force on the spinal rod.

Embodiments of the invention will now be described with reference to the Figures, wherein like numerals reflect like elements throughout. The terminology used in the description presented herein is not intended to be interpreted in any limited or restrictive way, simply because it is being utilized in conjunction with detailed description of certain specific embodiments of the invention. Furthermore, embodiments of the invention may include several novel features, no single one of which is solely responsible for its desirable attributes or which is essential to practicing the invention described herein. The words proximal and distal are applied herein to denote specific ends of components of the instrument described herein. A proximal end refers to the end of an instrument nearer to an operator of the instrument when the instrument is being used. A distal end refers to the end of a component further from the operator and extending towards the surgical area of a patient and/or the implant.

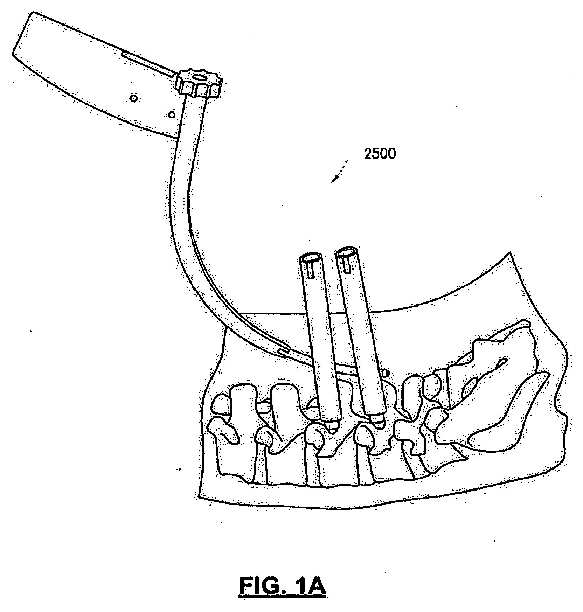

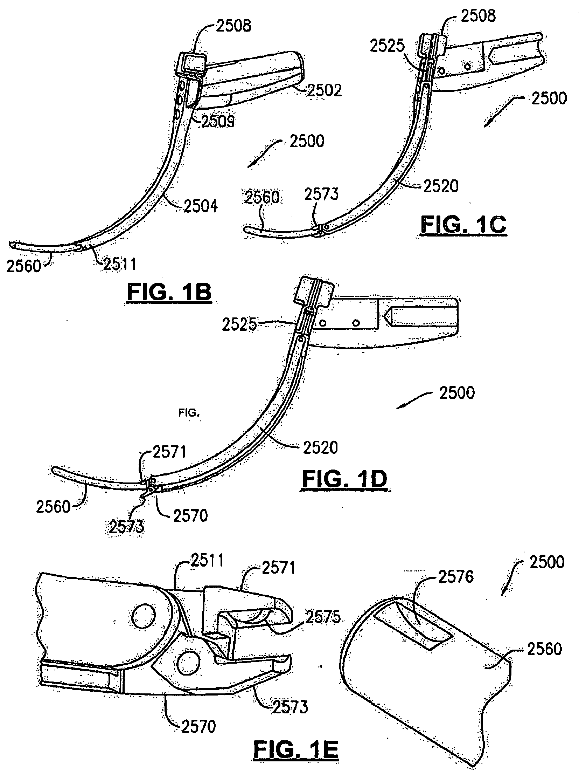

FIGS. 1a-e illustrate an exemplary percutaneous end-holding rod inserter tool 2500, according to some embodiments of the present invention. The tool 2500 allows rod holding during insertion of the rod through a screw extender system. The tool 2500 includes a handle 2502 and a curved shaft 2504. The shaft 2504 has a proximal end 2509 to which a control knob 2508 is attached and a distal end 2511 that is configured to secure a rod 2560. The proximal end 2509 is further configured to be permanently coupled to the handle 2502. The handle 2502 is configured to assist the user of the tool 2500 during rod-insertion procedures. The distal end 2511 includes a rod-holding mechanism 2570. The mechanism 2570 is configured to clamp the rod 2560 in a pliers-type fashion. The mechanism 2570 may include an upper immovable lip 2571 that is permanently coupled to the distal end of the shaft 2504 and a pivoting lip 2573 that is rotatably coupled to the distal end of the shaft 2504.

As illustrated in FIG. 1e, in some embodiments, the immovable lip 2571 can be configured to include a male mating feature 2575 that is configured to interact with a corresponding female mating feature 2576 disposed on the rod 2560. In some embodiments, the pivoting lip 2573 can be also configured to include similar mating features that can interact with corresponding mating features disposed on the rod 2560 (not shown in FIG. 1e). The mating features allow better gripping of the rod 2560, when the rod 2560 is placed between the immovable lip 2571 and the pivoting lip 2573. The mating features are also configured to prevent axial and torsion forces during rod-insertion procedures.

The curved shaft 2504 further includes a slidable rod 2520 disposed in an interior portion of the shaft 2504. The slidable rod 2520 is coupled to a threaded mechanism 2525 disposed at the proximal end of the shaft 2504. The slidable rod 2520 is further rotatably coupled to the pivoting lip 2573.

To secure the rod 2560 to the tool 2500, the knob 2508 is rotated in one direction (e.g., counterclockwise), causing the rod 2520 to be pushed away from the handle 2502 and thereby rotating the pivoting lip 2573 in a downward direction away from the immovable lip 2571. The rod 2560 is inserted into an opening created by the two lips 2571, 2573. In some embodiments, the mating features on the rod 2560 and the lip 2571 can interact with each other. Once the rod 2560 is inserted, the knob 2508 is rotated in an opposite direction (e.g., clockwise), thus causing the slidable rod 2520 to retract toward the handle, and thereby rotating the pivoting lip 2573 in an upward direction toward the immovable lip 2571 and the rod 2560. In some embodiments, the mating features of the rod 2560 and the pivoting lip 2573 can be configured to interact to further secure the rod 2560 inside the mechanism 2570. In some embodiments, the tool 2500 can be configured to have a stopper mechanism that can be configured to prevent accidental unwinding of the knob 2508, which can release the rod 2560. As can be understood by one skilled in the art, the tool 2500 can have both lips 2571, 2573 rotate to secure the rod.

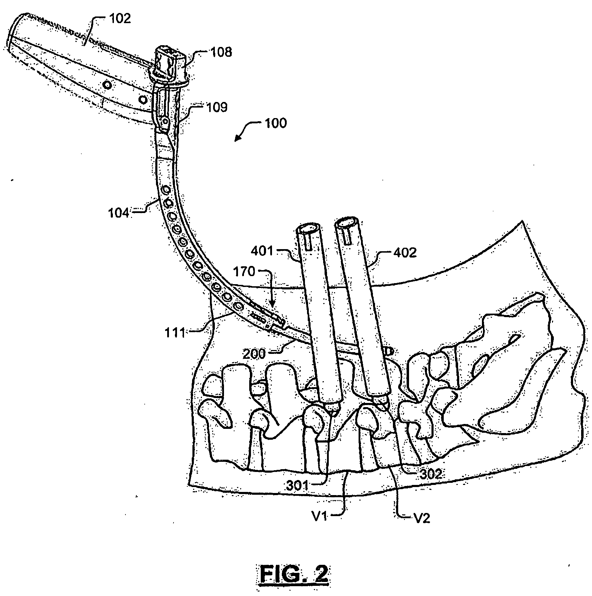

FIGS. 2-6 illustrate an improved exemplary inserter instrument 100 for percutaneous insertion of a spinal rod. The instrument 100 shares similar features as the instrument 2500 which are labeled with similar numbers. In practice, the instrument 100 may be used in a minimally invasive procedure to insert a rod 200 through a surgical incision in the body of a patient as illustrated in FIG. 2. For example, the patient may lie face down on a surgical table while a medical professional inserts two pedicle screws 301 and 302 into pedicles of adjacent vertebrae V1 and V2. Screw extenders 401 and 402 may be attached to the pedicle screws 301 and 302 and extend proximally through first and second minimally invasive surgical incisions in the body of the patient. The extenders 401 and 402 include channels, slots, or openings to receive the rod 200 and guide the rod into polyaxial heads of the screws 301 and 302. The instrument 100 grasps the rod 200 at one end such that the rod 200 is cantilevered towards the screw extenders 401 and 402. The curved shape of the instrument 100 and end-holding ability allows the user to introduce the rod 200 through one of the first or second incisions or an adjacent third incision and into slots of the extenders 401 and 402 and/or slots in heads of the screws 301 and 302.

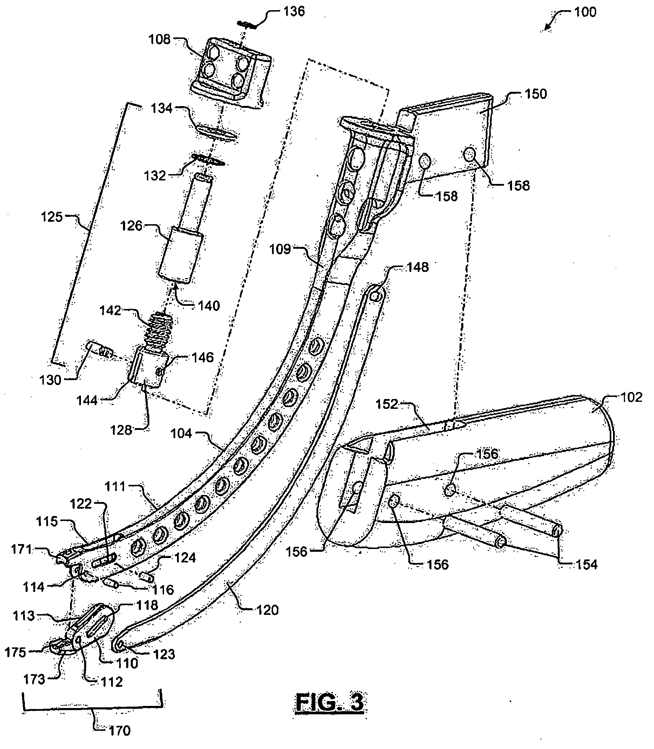

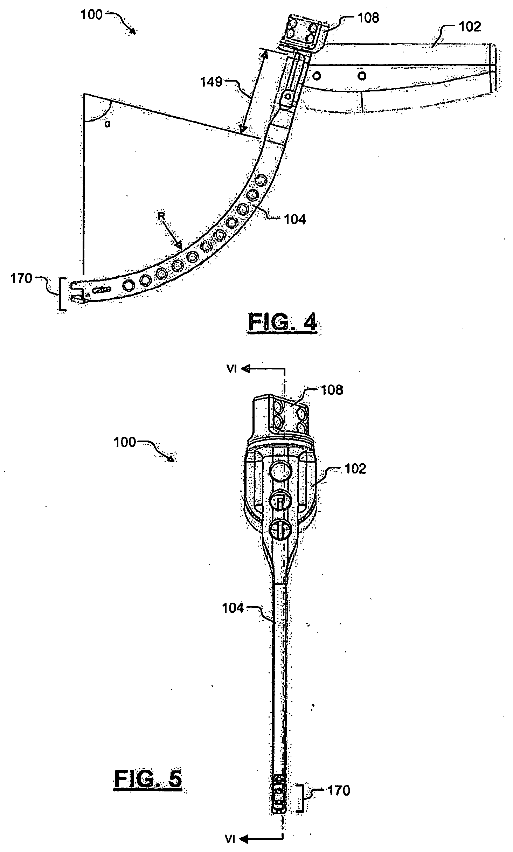

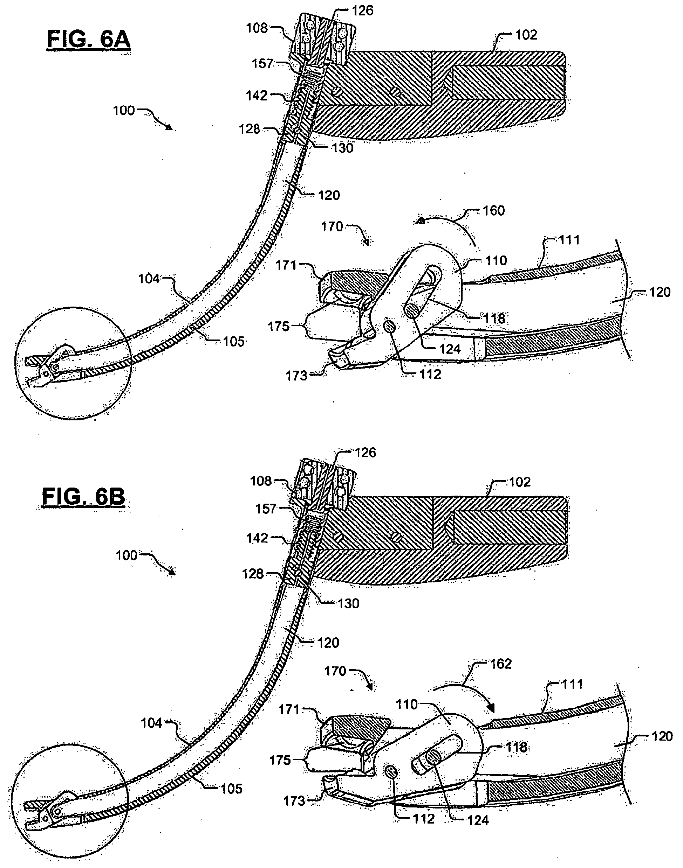

Referring now also to FIG. 3, the instrument 100 includes a handle 102 attached to a proximal end 109 of a curved housing 104. The curved housing 104 extends from the handle 102 to a distal end 111 and includes a channel or pathway 105 enclosed by the housing 104, that extends from the proximal end 109 to the distal end 111 as shown more clearly in FIGS. 6A-6D. The pathway 105 may include the same or similar curvature as the curved housing 104. In some examples, the curvature may include a 60-90 degree bend. In FIG. 4, the curvature or bend includes an angle a of approximately 66 degrees from the proximal end 109 to the distal end 111 of the housing 104. The curvature may include a radius R of curvature. The radius R may be any amount of curvature to ease insertion of the spinal rod.

The handle 102 may include a control knob 108 for adjusting various features of the instrument 100 as described in detail below. For example, as shown in FIG. 3, the instrument 100 may include a rod holding mechanism 170 at the distal end 111 comprising an immovable lip 171 and a pivoting lip 173 on a jaw piece 110. One or both of the two lips 171 and 173 may include a male mating feature 175 that couples with a female mating feature of the spinal rod 200 similar to the features male and female features 2575 and 2576 of the previous example. One skilled in the art would likewise recognize that the male and female style mating features could be switched from the lips to the rod for the same result. The instrument 100 further includes a slidable rod 120 that slides within the pathway 105 of the curved housing 104. The slidable rod 120 may include the same curved profile as the curved house 104. At a proximal end of the instrument 100, a threaded mechanism 125 may couple the slidable rod 120 to the control knob 108.

Continuing with FIG. 3, the exploded view illustrates various other features of the present invention that enable greater gripping forces and smoother actuation than prior instruments. In some examples, the instrument 100 may include various linkages between the control knob 108, slidable rod 120, and the jaw piece 110. At the proximal end of the instrument 100, the control knob 108 may function to position the slidable rod 120 within the pathway 105 and actuate the jaw piece 110. Rotating the control knob 108 in one direction may cause the jaw piece 110 to close and grasp a spinal rod. Rotating the control knob 108 in another direction may cause the jaw piece 110 to open and release the spinal rod.

At the distal end of the instrument 100, for example, the pivoting lip 173 may extend or project distally from the jaw piece 110. The jaw piece 110 couples with the distal end 111 of the housing 104 and a distal end of the slidable rod 120. The jaw piece 110 may include at a distal end a first pivot aperture 112 that aligns with a second pivot aperture 114 in the lateral sides of the housing 104 to receive a pivot pin 116. Thus the jaw piece 110 may rotate or pivot about a longitudinal axis of the pivot pin 116 as forces are applied on its proximal end. In some positions, the proximal end of the jaw piece 110 may project through an opening 115 in the proximal surface of the housing 104.

The jaw piece 110 may include at its proximal end a first guide slot 118 that aligns with a second guide slot 122 in one or both lateral sides of the housing 104 to receive a guide pin 124. The guide pin 124 may transfer force to the jaw piece 110 through engagement with and guidance by the guide slots 118 and 122. The slidable rod 120 also may include a guide aperture 123 at its distal end that receives the guide pin 124. For example, the proximal end of the jaw piece 110 may couple with the distal end of the slidable rod 120 by the guide pin 124. The jaw piece 110 may include an opening 113 for receiving the distal end of the slidable rod 120. As the jaw piece 110 pivots about the pivot pin 116, the spinal rod may be grasped or released. Interaction of the guide pin 124 with the first and second guide slots 118 and 122 forces the proximal end of the jaw piece 110 to rotate.

The threaded mechanism 125 may include various features within the proximal end 109 of the housing 104 that permit rotation by the control knob 108 to extend and retract the slidable rod 120. For example, the threaded mechanism 125 may include a first shaft 126, a second shaft 128, a drive pin 130, a first retainer 132, a washer 134, and a second retainer 136. A proximal end of the first shaft 126 may be fixed to the control knob 108 by the retainers 132 and 136 with the washer 134 therebetween. The washer 134 may reduce friction between the control knob 108 and the proximal end 109 of the housing 104. The retainers 132 and 136 may retain the first shaft 126 within an opening 138 of the proximal end 109 of the housing 104. The first shaft 126 may be rotated within the housing 104 by the control knob 108. A distal end of the first shaft 126 may include receptacle 140 for receiving the second shaft 128. For example, the receptacle 140 may include an internal thread (not shown) that engages an external thread 142 of the second shaft 128. As the control knob 108 turns, the external thread 142 engages the internal thread to retract or advance the second shaft 128 proximally or distally as illustrated in FIGS. 6A-6D.

Continuing with the threaded mechanism 125 in FIG. 3, the second shaft 128 may couple to the proximal end of the slidable rod 120 by the drive pin 130. For example, the proximal end of the slidable rod 120 may be captured by an opening 144 on the distal end of the second shaft 128. The drive pin 130 may pass through first drive apertures 146 in the second shaft 128 and a second drive aperture 148 in the slidable rod 120 to couple the second shaft 128 with the slidable rod 120. Thus, as the control knob 108 rotates the slidable rod 120 moves with the second shaft 128 either proximally or distally within the housing 104.

For example, as the control knob 108 rotates in one direction, such as a clockwise direction, proximal movement of the slidable rod 120 pulls the guide pin 124 within the guide aperture 123 proximally in the guide slots 118 and 122. As the guide pin 124 advances proximally, the jaw piece 110 is forced to rotate about the pivot pin 116 due to the travel of the guide pin 124 within the slots 118 and 122. Rotation of the jaw piece 110 positions the pivoting lip 173 closer to the upper lip 171. Likewise, as the control knob 108 rotates in another direction, such as the opposite counter-clockwise direction, distal movement of the slidable rod 120 pushes the guide pin 124 within the guide aperture 123 distally in the guide slots 118 and 122. As the guide pin advances distally, the jaw piece 110 is forced to rotate about the pivot pin 116 due to the travel of the guide pin 124 within the slots 118 and 122. Rotation of the jaw piece 110 positions the pivoting lip 173 further from the upper lip 171.

The handle 102 may be coupled to the proximal end 109 of the housing 104 in various manners. Continuing with FIG. 3 and also FIGS. 4 and 5, the handle 102, for example, may extend from the proximal end 109 at some angle. The proximal end 109 may include a substantially straight portion 149 that contains the threaded mechanism 125. The handle 102 may extend from the straight portion 149 at approximately a right angle or slightly more. The proximal end 109 may include an attachment portion 150 that fits within a mating recessed portion 152 of the handle 102. One or more fasteners 154 may extend through apertures 156 in the handle 102 and apertures 158 in the attachment portion 150 to secure the handle 102 to the housing 104.

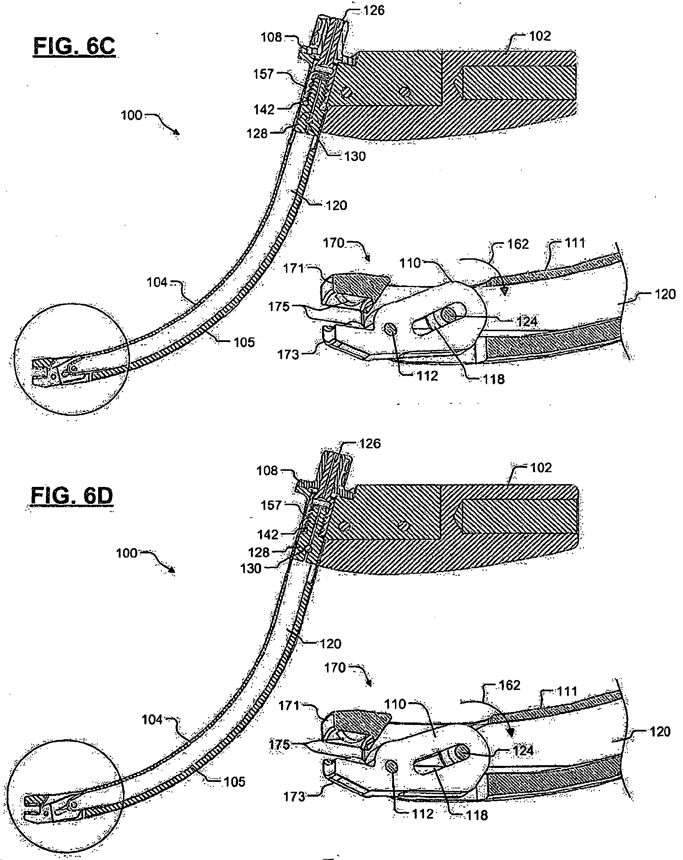

FIGS. 6A-6D are cross-sectional views of the instrument 100 in a series of positions in a plane VI as shown in FIG. 5. In FIG. 6A, the instrument 100 is shown in a first position, such as a fully extended or a fully open position. In the first position, the rod-holding mechanism 170 is fully open to receive a rod 200 as shown in FIG. 2. In FIG. 6B, the instrument 100 is shown in second position, such as a partially retracted or partially closed position. In the second position, the rod-holding mechanism 170 begins to close to grasp the rod. In FIG. 6C, the instrument 100 is shown in a third position, such as a mostly retracted or mostly closed position. In the third position, the rod-holding mechanism 170 may begin to engage the rod. In FIG. 6D, the instrument 100 is shown in a fourth position, such as a fully retracted or fully closed position. In the fourth position, the rod-holding mechanism 170 may apply significant force to the end of the rod.

Referring now to FIG. 6A, the instrument 100 is in the first position with the slidable rod 120 fully extended towards the distal end 111 of the housing 104. For example, the knob 108 may be rotated until most of internal thread 157 inside the first shaft 126 of the threaded mechanism 125 disengages most of the external thread 142 on the second shaft 128. Thus, the drive pin 130 within the first drive apertures 146 pushes on the slidable rod 120 via the second aperture 148. At the distal end of the slidable rod 120, the guide pin 124 pushes distally on the slot 118 within the jaw piece 110 causing rotation in the direction of arrow 160 until the instrument 100 is fully open and the slidable rod 120 is fully extended. The jaw piece 110 may rotate or pivot about the pivot pin 112 as the slidable rod 120 pushes the guide pin 124 in the slots 118 and 122. In the fully open position, the jaw piece 110 may provide an opening for receiving the rod 200. A medical professional may then position the rod between the upper and lower mating features 175 of the lips 171 and 173.

Continuing now with FIGS. 6B and 6C, the instrument 100 may move to the second position and third position as the slidable rod 120 is pulled proximally. For example, the control knob 108 may be rotated to engage more of the internal thread 157 inside the first shaft 126 with the external thread 142 on the second shaft 128. Thus, the drive pin 130 within the first drive apertures 146 begins to pull on the slidable rod 120 via the second drive aperture 148. At the distal end of the slidable rod 120, the guide pin 124 pulls proximally on the slot 118 within the jaw piece 110 causing rotation in the direction of arrow 162 until the instrument 100 begins to partially close and the slidable rod 120 is partially retracted. The jaw piece 110 may rotate or pivot about the pivot pin 112 as the slidable rod 120 pulls the guide pin 124 in the guide slots 118 and 122. In the partially closed position, the jaw piece 110 may begin to close causing the mating features 175 to come closer to engagement with the rod. The knob 108 may continue to be rotated to engage more of the internal thread 157 with the external thread 142 and thus advancing the slidable rod 120 further proximally. Thus the jaw piece 110 may rotate until the instrument 100 begins to be mostly close and the slidable rod 120 is mostly retracted.

In FIG. 6D, the instrument 100 is in a fourth position with the slidable rod 120 fully retracted towards the proximal end 109 of the housing 104. For example, the control knob 108 may be rotated until most of the internal thread 157 inside the first shaft 126 engages most of the external thread 142 on the second shaft 128. Thus, the drive pin 130 within the first drive apertures 146 pulls on the slidable rod 120 via the second drive aperture 148. At the distal end of the slidable rod 120, the guide pin 124 pulls proximally on the guide slot 118 within the jaw piece 110 causing rotation in the direction of arrow 162 until the instrument 100 is in the closed position. The jaw piece 110 may rotate or pivot about the pivot pin 112 as the slidable rod 120 continues to pull the guide pin 124 in the guide slots 118 and 122. In the closed position, the jaw piece 110 may apply significant force to grasp an end of a rod between the upper lip 171 and the lower lip 173 and allow a medical professional to maneuver the rod during a surgical procedure.

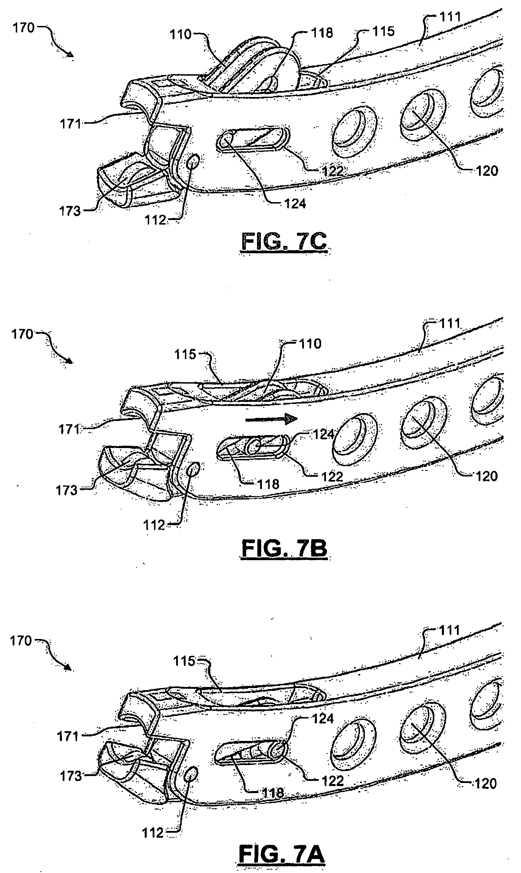

FIGS. 7A-7C illustrate another view of the instrument 100 with the rod-holding mechanism 170 in various positions. These figures more clearly show how the guide pin 124 and guide slots 118 and 122 interact to control motion of the jaw piece 110 and pivoting lip 173. For example, in FIG. 7A, the slidable rod 120 may be fully advanced distally forcing the guide pin 124 to the distal end of the second guide slot 122. The guide pin 124 provides a moment about the pivot pin 112 to rotate the jaw piece 110 into the fully open position. Continuing with FIG. 7B, the slidable rod 120 may be advanced proximally to pull the guide pin 124 within the second guide slot 122. The guide pin 124 provides a moment about the pivot pin 112 to begin to rotate the jaw piece 110. In FIG. 7C, the slidable rod 120 advances may be fully advanced proximally forcing the guide pin to the proximal end of the slot 122. The guide pin 124 provides a moment about the pivot pin 112 to rotate the jaw piece 110 into the fully closed position.

Example embodiments of the methods and systems of the present invention have been described herein. As noted elsewhere, these example embodiments have been described for illustrative purposes only, and are not limiting. Other embodiments are possible and are covered by the invention. Such embodiments will be apparent to persons skilled in the relevant art(s) based on the teachings contained herein. Thus, the scope of the present invention should not be limited by any of the above-described exemplary embodiments, but should be defined only in accordance with the following claims and their equivalents.

## Claims

1. An instrument (100) for grasping an end of a spinal rod (200) and percutaneous insertion of the spinal rod, comprising:
a curved housing (104) extending from a proximal end (109) to a distal end (111) and including an interior channel (105);
an immovable lip (171) extending from the distal end;
a first slot (122) in a side wall of the distal end;
a pivoting lip (110, 173) rotatably coupled to the distal end opposite the immovable lip; and
a slidable rod (120) disposed within the interior channel and slidably coupled at a first end to the first slot and a second slot (118) of the pivoting lip such that the slidable rod is translatable within the interior channel to position the pivoting lip relative to the immovable lip to hold a spinal rod therebetween.

2. The instrument of claim 1, further comprising a threaded mechanism (125) within a portion of the proximal end rotatably engaged with a second end of the slidable rod and arranged to translate the slidable rod within the interior channel.

3. The instrument of claim 1 or claim 2, wherein at least one of the immovable lip and the pivoting lip includes at least one mating feature configured to interact with at least one corresponding mating feature disposed on a spinal rod to further secure the rod.

4. The instrument of claim 3, wherein the at least one mating feature of the at least one of the immovable lip and the pivoting lip comprises a male feature for engaging a corresponding female feature of a spinal rod.

5. The instrument of any preceding claim, wherein the immovable lip and the pivoting lip can be positioned to hold the spinal rod at a predetermined angle with respect to the curved housing.

6. The instrument of any preceding claim, wherein the curved housing includes a groove and the slidable rod includes a corresponding protrusion configured to translate inside the groove to prevent excessive translation of the sliding rod.

7. The instrument of any of claims 2 to 6, further comprising a rotatable knob (108) coupled to the threaded mechanism.

8. The instrument of claim 7, configured such that rotation of the knob in one direction translates the slidable rod toward the distal end of the curved housing to secure a rod between the pivoting lip and the immovable lip.

9. The instrument of claim 8, configured such that rotation of the knob in another direction translates the slid able rod away from the distal end of the curved housing to release the rod.

10. The instrument of any of claims 2 to 9, wherein the threaded mechanism includes a second shaft (128) rotatably coupled with the curved housing and a first shaft (126) in threaded engagement with the second shaft.

11. The instrument of any of claims 2 to 9, wherein a portion (126, 132, 134, 136) of the threaded mechanism is arranged to rotate relative to the slidable rod.

12. The instrument of any preceding claim, further comprising a guide pin (124) extending through the first slot and the second slot to guide the movement of the pivoting lip as the slidable rod translates within the housing.

13. The instrument of claim 12, wherein the distal end comprises a third slot in an opposite side wall to the first slot and wherein the guide pin further extends through the third slot.

14. The instrument of any preceding claim, further comprising a pivot pin (116) extending through the pivoting lip and the distal end to rotatably couple the pivoting lip to the distal end.

15. The instrument of any preceding claim, wherein the distal end comprises an opening (115) through which a portion of the pivoting lip projects in some positions.

## Patentansprüche

1. Instrument (100) zum Greifen eines Endes eines Wirbelsäulenstabes (200) und zum perkutanen Einführen des Wirbelsäulenstabes, umfassend:
ein gebogenes Gehäuse (104), das sich von einem proximalen Ende (109) zu einem distalen Ende (111) erstreckt und einen inneren Kanal (105) aufweist;
eine unbewegliche Lippe (171), die sich von dem distalen Ende erstreckt;
einen ersten Schlitz (122) in einer Seitenwand des distalen Endes;
eine schwenkbare Lippe (110, 173), die drehbar an dem distalen Ende gegenüber der unbeweglichen Lippe befestigt ist; und
eine verschiebbare Stange (120), die innerhalb des inneren Kanals angeordnet und verschiebbar an einem ersten Ende mit dem ersten Schlitz und einem zweiten Schlitz (118) der schwenkbaren Lippe derart gekoppelt ist, dass die verschiebbare Stange translatorisch in dem inneren Kanal beweglich ist, um die schwenkbare Lippe relativ zu der unbeweglichen Lippe zu positionieren, um eine Wirbelsäulenstange dazwischen zu halten.

2. Instrument nach Anspruch 1, ferner umfassend einen Gewindemechanismus (125) in einem Abschnitt des proximalen Endes, das drehbar mit einem zweiten Ende der verschiebbaren Stange in Eingriff steht, und angeordnet ist, die verschiebbare Stange in dem inneren Kanal translatorisch zu bewegen.

3. Instrument nach Anspruch 1 oder Anspruch 2, wobei mindestens eine von der unbeweglichen Lippe und der schwenkbaren Lippe mindestens ein Zusammenfügungsmerkmal aufweist, das konfiguriert ist, um mit mindestens einem entsprechenden Zusammenfügungsmerkmal, das an einem Wirbelsäulenstab angeordnet ist, zusammenzuwirken, um den Stab weiter zu sichern.

4. Instrument nach Anspruch 3, wobei das mindestens eine Zusammenfügungsmerkmal von dem mindestens einen von der unbeweglichen Lippe und der schwenkbaren Lippe ein Einsteckmerkmal zum Eingriff mit einem entsprechenden Aufnahmemerkmal einer Wirbelsäulenstange aufweist.

5. Instrument nach einem der vorhergehenden Ansprüche, wobei die unbewegliche Lippe und die schwenkbare Lippe positioniert werden können, um die Wirbelsäulenstange in einem vorbestimmten Winkel in Bezug auf das gebogene Gehäuse zu halten.

6. Instrument nach einem der vorhergehenden Ansprüche, wobei das gebogene Gehäuse eine Rille aufweist und die verschiebbare Stange einen entsprechenden Vorsprung aufweist, der konfiguriert ist, sich innerhalb der Rille translatorisch zu bewegen, um eine übermäßige translatorische Bewegung der verschiebbaren Stange zu verhindern.

7. Instrument nach einem der Ansprüche 2 bis 6, ferner umfassend einen drehbaren Knopf (108), der mit dem Gewindemechanismus gekoppelt ist.

8. Instrument nach Anspruch 7, das derart konfiguriert ist, dass eine Drehung des Knopfes in eine Richtung die verschiebbare Stange in Richtung des distalen Endes des gebogenen Gehäuses translatorisch bewegt, um einen Stab zwischen der schwenkbaren Lippe und der unbeweglichen Lippe zu sichern.

9. Instrument nach Anspruch 8, das derart konfiguriert ist, dass eine Drehung des Knopfes in eine andere Richtung die verschiebbare Stange von dem distalen Ende des gebogenen Gehäuses translatorisch wegbewegt, um die Stange freizugeben.

10. Instrument nach einem der Ansprüche 2 bis 9, wobei der Gewindemechanismus einen zweiten Schaft (128), der drehbar mit dem gebogenen Gehäuse gekoppelt ist, und einen ersten Schaft (126) in Gewindeeingriff mit dem zweiten Schaft aufweist.

11. Instrument nach einem der Ansprüche 2 bis 9, wobei ein Abschnitt (126, 132, 134, 136) des Gewindemechanismus angeordnet ist, sich relativ zu der verschiebbaren Stange zu drehen.

12. Instrument nach einem der vorhergehenden Ansprüche, ferner umfassend einen Führungsstift (124), der sich durch den ersten Schlitz und den zweiten Schlitz erstreckt, um die Bewegung der schwenkbaren Lippe zu führen, wenn sich die verschiebbare Stange in dem Gehäuse translatorisch bewegt.

13. Instrument nach Anspruch 12, wobei das distale Ende einen dritten Schlitz in einer dem ersten Schlitz gegenüberliegenden Seitenwand aufweist und wobei der Führungsstift sich ferner durch den dritten Schlitz erstreckt.

14. Instrument nach einem der vorhergehenden Ansprüche, ferner umfassend einen Schwenkzapfen (116), der sich durch die schwenkbare Lippe und das distale Ende erstreckt, um die schwenkbare Lippe drehbar an das distale Ende zu koppeln.

15. Instrument nach einem der vorhergehenden Ansprüche, wobei das distale Ende eine Öffnung (115) aufweist, durch die ein Abschnitt der schwenkbaren Lippe in einigen Positionen hervorragt.

## Revendications

1. Instrument (100) permettant la saisie d'une extrémité d'une tige vertébrale (200) et l'insertion percutanée de la tige vertébrale, comprenant :
un logement incurvé (104) s'étendant d'une extrémité proximale (109) vers une extrémité distale (111) et comprenant un canal intérieur (105) ;
un bord fixe (171) s'étendant depuis l'extrémité distale ;
une première fente (122) dans une paroi latérale de l'extrémité distale ;
un bord pivotant (110, 173) accouplé de manière rotative à l'extrémité distale en face du bord fixe ;
et
une tige coulissante (120) disposée à l'intérieur du canal intérieur et accouplée de manière coulissante au niveau d'une première extrémité à la première fente et une deuxième fente (118) du bord pivotant de sorte que l'extrémité coulissante puisse effectuer un mouvement de translation à l'intérieur du canal intérieur pour positionner le bord pivotant par rapport au bord fixe pour maintenir une tige vertébrale entre eux.

2. Instrument selon la revendication 1, comprenant en outre un mécanisme fileté (125) à l'intérieur d'une partie de l'extrémité proximale en prise de manière rotative avec une seconde extrémité de la tige coulissante et agencé pour translater la tige coulissante à l'intérieur du canal intérieur.

3. Instrument selon la revendication 1 ou la revendication 2, dans lequel le bord fixe et/ou le bord pivotant comprend/comprennent au moins un élément d'accouplement configuré pour interagir avec au moins un élément d'accouplement correspondant disposé sur une tige vertébrale pour une meilleure fixation de la tige.

4. Instrument selon la revendication 3, dans lequel le ou les élément(s) d'accouplement du bord fixe et/ou du bord pivotant comprend/comprennent un élément mâle destiné à entrer en prise avec un élément femelle correspondant d'une tige vertébrale.

5. Instrument selon l'une quelconque des revendications précédentes, dans lequel le bord fixe et/ou le bord pivotant peut/peuvent être positionné(s) pour maintenir la tige vertébrale en formant un angle prédéterminé avec le logement incurvé.

6. Instrument selon l'une quelconque des revendications précédentes, dans lequel le logement incurvé comprend une rainure et la tige coulissante comprend une saillie correspondante configurée pour translater à l'intérieur de la rainure pour empêcher une translation excessive de la tige coulissante.

7. Instrument selon l'une quelconque des revendications 2 à 6, comprenant en outre un bouton rotatif (108) accouplé au mécanisme fileté.

8. Instrument selon la revendication 7, configuré de sorte que la rotation du bouton dans une direction translate la tige coulissante en direction de l'extrémité distale du logement incurvé pour fixer une tige entre le bord pivotant et le bord fixe.

9. Instrument selon la revendication 8, configuré de sorte que la rotation du bouton dans une autre direction translate la tige coulissante depuis l'extrémité distale du logement incurvé pour libérer la tige.

10. Instrument selon l'une quelconque des revendications 2 à 9, dans lequel le mécanisme fileté comprend un second arbre (128) accouplé de manière rotative au logement incurvé et un premier arbre (126) en prise filetée avec le second arbre.

11. Instrument selon l'une quelconque des revendications 2 à 9, dans lequel une partie (126, 132, 134, 136) du mécanisme fileté est agencée pour entrer en rotation par rapport à la tige coulissante.

12. Instrument selon l'une quelconque des revendications précédentes, comprenant en outre un axe de guidage (124) s'étendant à travers la première fente et la deuxième fente pour guider le mouvement du bord pivotant lorsque la tige coulissante translate à l'intérieur du logement.

13. Instrument selon la revendication 12, dans lequel l'extrémité distale comprend une troisième fente dans une paroi latérale opposée à la première fente et dans lequel l'axe de guidage s'étend davantage à travers la troisième fente.

14. Instrument selon l'une quelconque des revendications précédentes, comprenant en outre un axe de pivotement (116) s'étendant à travers le bord pivotant et l'extrémité distale pour accoupler de manière rotative le bord pivotant à l'extrémité distale.

15. Instrument selon l'une quelconque des revendications précédentes, dans lequel l'extrémité distale comprend une ouverture (115) à travers laquelle une partie du bord pivotant fait saillie dans certaines positions.
